# FASCICULE DE BREVET EUROPEEN

(11) **EP 1 977 712 B1**
(45) Date de publication et mention de la délivrance du brevet: **21.07.2010**
(21) Numéro de dépôt: 07300937.5
(22) Date de dépôt: 06.04.2007
(51) Int. Cl.: A61B 19/00, A61M 16/00, A61G 12/00

(54) **Unité fonctionnelle hospitalière mobile pour la distribution momentanée de fluides médicaux**
Mobile Krankenhausfunktionseinheit zur augenblicklichen Verteilung von medizinischen Flüssigkeiten
Mobile functional hospital unit for temporarily dispensing medical fluids

(43) Date de publication de la demande: 08.10.2008
(73) Titulaire: Air Liquide Sanita Services SpA, 20148 Milano (IT)
(72) Inventeur: Denando, Vittorio, 20019 Settimo Milanese (MI) (IT); Deneux, Patrick, 20100 Milano (IT); Demerville, Nathalie, 20030 Bovisio Masciago (IT)
(74) Mandataire: Pittis, Olivier

(56) Documents cités:
- EP-A2- 0 464 410
- WO-A-2004/084981
- WO-A-2006/074473
- US-A- 3 428 383
- US-A- 5 337 845
- SFAR: "Une aide à la lecture pour l'arrêté du 3 octobre 1995 relatif aux modalités d'utilisation et de contrôle des matériels et dispositifs médicaux" INTERNET ARTICLE, [Online] 1996, XP002452241 Extrait de l'Internet: URL:http://www.sfar.org/dispositifs.html> [extrait le 2007-09-25]
- TAEMA: "Anaesthesia tray Clarys 2000" INTERNET ARTICLE, [Online] 6 novembre 2001 (2001-11-06), XP002452242 Extrait de l'Internet: URL:http://www.taema.com/en/business/hopit al/CLARYS2000_%20%20GB.pdf> [extrait le 2007-09-25]
- TAEMA: "Anaesthesia tray Felix Visio" INTERNET ARTICLE, [Online] 29 avril 2004 (2004-04-29), XP002452243 Extrait de l'Internet: URL:http://www.taema.com/en/business/hopit al/FelixVisio.pdf> [extrait le 2007-09-25]
- DRAEGER: "Anesthesia tray Zeus" INTERNET ARTICLE, [Online] 13 novembre 2006 (2006-11-13), XP002452244 Extrait de l'Internet: URL:http://www.draeger.com/MT/internet/pdf /lib/de/anesth_work/zeus_br.pdf> [extrait le 2007-09-25]

## Description

La présente invention concerne une unité fonctionnelle de distribution momentanée de fluides, c'est-à-dire de gaz et de vide, et de services annexes dans l'environnement hospitalier, en particulier une unité fonctionnelle de secours ou d'assistance, destinée à rétablir momentanément la fonction de distribution de gaz ou de vide, et de services annexes au cas où elle défaillerait de manière imprévue ou inopinée au sein d'un bâtiment hospitalier ou similaire.

Dans l'environnement hospitalier, par exemple en salles d'opération, dans les locaux de soins d'urgence, de réanimation ou d'hospitalisation, on connaît la grande importance que revêt la disponibilité de fluides médicaux (oxygène, air, vide...) pour leur utilisation programmée à moyen terme et à long terme ainsi que pour des interventions d'urgence.

On sait également que, pour cette raison, on prévoit des installations appropriées de distribution, structurées et équipées de manière à rendre ces fluides médicaux disponibles sur plusieurs "prises murales" respectives ou unités terminales d'utilisation, installées rationnellement là où elles doivent servir, par exemple en salles de soins ou dans les chambres.

Ces prises ou unités terminales d'utilisation agencées sur les murs ou sur des suspensions fixes ou mobiles, se présentent habituellement sous la forme de « buses » ou de prises de raccordement qui peuvent être raccordées au moyen de connecteurs spéciaux et de conduits flexibles aux appareils médicaux qui utilisent les fluides médicaux, par exemple des masques à oxygène, des dispositifs d'assistance à la respiration ou à la ventilation, des dispositifs d'aspiration des fluides corporels, et des appareils similaires.

On sait par ailleurs que la régularité de la distribution des fluides médicaux peut subir des interruptions accidentelles et imprévisibles, par exemple à cause de dommages causés aux conduits d'alimentation en fluides, aux organes de commande, de régulation et d'interruption de l'écoulement des différents gaz en circulation.

Pour tenter d'affronter ces situations d'urgence, en plus d'adopter des systèmes de distribution "sécurisés" selon ce que prévoient les normes en vigueur, il est d'usage de constituer des réserves de gaz médicaux qui sont structurellement et fonctionnellement indépendantes des installations de distribution, en les chargeant en général en bouteilles de gaz munies d'organes adéquats de distribution et de contrôle du gaz.

Cependant, l'utilisation de bouteilles ou bonbonnes de gaz présente de nombreux inconvénients, liés surtout à leur manipulation et à la possibilité qu'elles subissent des chocs accidentels, telles des chutes, avec risque de rupture ou de déformation des organes de distribution et de contrôle dont elles sont dotées et, de là, avec une réduction plus ou moins importante de leur fonctionnalité.

En outre, une grande partie des bouteilles de gaz gardées en réserve ont le défaut d'être relativement lourdes et de dimensions importantes, et ne sont dès lors pas commodes à utiliser en cas d'urgence, même par du personnel spécialisé. On sait en effet que les situations de défaut d'alimentation peuvent créer des états de panique et amènent les opérateurs, même les plus expérimentés, à travailler dans des conditions de stress qui peuvent conduire à des erreurs ou à des blessures avec les matériels actuels.

En outre, lorsque l'alimentation de tous les gaz médicaux est subitement interrompue, il est nécessaire de rétablir toutes les alimentations avec une importante mise en oeuvre et une haute probabilité de commettre des erreurs, du fait que chaque bouteille contient un gaz différent.

Il faut également souligner le fait que, lorsque l'on utilise ces bouteilles de gaz de réserve uniquement de manière occasionnelle, il existe un risque potentiel qu'elles se déchargent au fil du temps et/ou qu'elles ne soient pas suffisamment chargées en gaz au moment précis où il est nécessaire de les employer.

De plus, il n'existe actuellement aucune solution totalement satisfaisante pour remédier à une interruption inopinée de la distribution centralisée de vide, c'est-à-dire à une mise hors service du réseau de vide servant à l'aspiration des cavités internes des patients, par exemple lors d'une intervention chirurgicale, à l'exception de certains systèmes électriques qui sont toutefois de puissance limitée, tels des aspirateurs électriques individuels.

Au vu de cela, un problème à résoudre est donc de pouvoir remédier totalement ou au moins en partie auxdits inconvénients de l'art antérieur en permettant, dans l'environnement hospitalier, la formation d'une réserve importante de gaz médicaux et de services annexes qui soit complètement indépendante d'une éventuelle installation de distribution classique de telle sorte qu'elle soit rapidement disponible en un point quelconque dudit environnement hospitalier.

Un autre objet de l'invention vise à proposer une solution particulièrement fiable et pouvant être mise en oeuvre efficacement dans les situations d'urgence en évitant les difficultés éventuelles liées au caractère sporadique de son utilisation.

De plus, un autre objet de l'invention est d'éviter au personnel médical et/ou paramédical, des opérations manuelles sur des bouteilles relativement dangereuses et auxquelles leurs fonctions normales ne les ont pas accoutumés de manière appropriée.

En outre, la solution de l'invention doit pouvoir informer immédiatement les opérateurs sur la disponibilité effective du produit, sur le niveau effectif de pression présent dans les bouteilles de gaz de réserve et dans le réseau de distribution secondaire.

Une solution selon la présente invention repose sur une unité fonctionnelle d'assistance mobile montée sur roues, pour l'alimentation en gaz médicaux et en vide, laquelle unité peut être déplacée immédiatement et facilement et est rendue fonctionnelle par les opérateurs du secteur hospitalier en cas d'interruption imprévue de la distribution normale de gaz médicaux, pour l'évacuation ou le transport des patients hors du service et pour la préparation rapide de zones de traitement des patients en cas de sortie en position couchée.

Plus précisément, l'invention porte sur une unité fonctionnelle hospitalière mobile pour la distribution momentanée de fluides médicaux, ladite unité étant munie de roues pour permettre un déplacement de l'unité sur le sol et comprenant :
- une armoire comportant une paroi frontale, une paroi arrière, des parois latérales, un sommet et un fond, et dont l'intérieur définit au moins un compartiment qui loge une ou plusieurs bouteilles de gaz médical et au moins une pompe d'aspiration du vide médical,
- au moins une prise arrière de prélèvement de fluide médical, c'est-à-dire de gaz ou de vide, conçue et adaptée pour être connectée à un réseau de distribution de fluides médicaux agencé au sein d'un bâtiment hospitalier et étant associée à la paroi arrière de ladite armoire, c'est-à-dire que la ou les prises arrière sont aptes à être connectées directement sur le réseau de canalisation de fluide qui parcours le bâtiment,
- au moins une prise latérale de prélèvement de fluide médical étant associée à au moins une paroi latérale de ladite armoire,
- au moins une ligne de raccordement relie fluidiquement au moins une bouteille de gaz à au moins l'une desdites prises arrière et latérale de prélèvement de gaz pour alimenter au moins une desdites prises arrière et latérale avec du gaz délivré par au moins une bouteille de gaz, et qui desservent l'unité, et
- des moyens de surveillance et de pilotage conçus pour et aptes à fournir à un utilisateur au moins une information relative à un dysfonctionnement de l'unité.

Selon le cas, l'unité fonctionnelle de l'invention peut comprendre l'une ou plusieurs des caractéristiques suivantes :
- elle comprend au moins deux bouteilles, de préférence deux paires de bouteilles de gaz.
- elle comprend au moins une paire de bouteilles contenant un premier gaz médical, de préférence de l'oxygène, et au moins une deuxième paire de bouteilles contenant un deuxième gaz médical différent du premier gaz médical, de préférence de l'air médical.
- les deux bouteilles d'une paire de bouteilles de gaz sont raccordées à une ligne de raccordement commune reliant les deux bouteilles de ladite paire de bouteilles à au moins l'une desdites prises arrière et latérale de prélèvement de gaz.
- les deux bouteilles d'une paire de bouteilles de gaz sont raccordées à une ligne de raccordement commune reliant lesdites deux bouteilles de ladite paire de bouteilles à au moins une prise arrière de prélèvement de gaz située sur la paroi arrière de ladite armoire, et à au moins une prise latérale de prélèvement de gaz située sur une paroi latérale de ladite armoire.
- une électrovanne est prévue sur la ou chaque ligne de raccordement, de préférence une ou les électrovannes sont commandées par les moyens de surveillance et de pilotage.
- au moins une prise arrière et/ou au moins une prise latérale de prélèvement de fluide médical est une prise d'aspiration de vide, et au moins une prise arrière et/ou au moins une prise latérale de prélèvement de fluide médical est une prise de distribution de gaz, en particulier d'oxygène ou d'air médical.
- sur la ou chaque ligne de raccordement sont prévus, agencés en série, entre la ou les bouteilles de gaz et la ou les prises de raccordement correspondantes, un détendeur de pression, une soupape anti-retour et une électrovanne, de préférence l'électrovanne est normalement ouverte.
- au moins une prise arrière et au moins une prise latérale sont des prises d'aspiration de vide reliées fluidiquement, via au moins une ligne de vide, à l'entrée d'aspiration de la pompe d'aspiration de manière à pouvoir créer une dépression au niveau desdites prises.
- elle comprend au moins une prise latérale de raccordement et au moins une prise arrière de raccordement pour un premier type de gaz médical, en particulier de l'oxygène, et au moins une prise latérale de raccordement et au moins une prise arrière de raccordement pour un deuxième type de gaz médical, en particulier de l'air médical.
- les prises de raccordement latérales sont dupliquées sur les deux parois latérales opposées de l'armoire.
- la paroi frontale de ladite armoire comporte un panneau de commande, de préférence ledit panneau de commande comprend un clavier et/ou un afficheur coopérant avec les moyens de surveillance et de pilotage.
- au moins une des parois latérales comporte une porte donnant accès au compartiment interne contenant la ou les bouteilles ou au compartiment interne contenant la pompe, de préférence au moins une porte est aménagée sur chacune des parois latérales.
- elle comprend une batterie d'alimentation électrique de secours conçue pour être apte à alimenter en courant électrique, les moyens de surveillance et de pilotage et/ou la ou les électrovannes, notamment en cas de défaillance du réseau de distribution électrique.
- elle comprend des poignées aménagées sur l'une ou plusieurs des parois latérales frontale ou arrière.
- le sommet de l'armoire forme une cuvette porte-objets.
- l'intérieur de l'armoire comporte au moins un compartiment principal logeant une ou plusieurs bouteilles de gaz médical et au moins un compartiment supplémentaire dans lequel est agencée au moins la pompe d'aspiration, lesdits compartiments étant munis chacun d'une ou plusieurs portes d'accès, de préférence le compartiment supplémentaire est fermé par une porte munie de fentes d'aération.
- au moins une prise arrière de prélèvement de fluide médical gaz est agencée au niveau de l'extrémité supérieure de la paroi arrière et à proximité du sommet et/ou au moins une prise latérale de prélèvement de fluide médical est agencée au niveau de l'extrémité supérieure d'au moins une paroi latérale et à proximité du sommet.
- au moins une partie supérieure de la face frontale est inclinée par rapport à un plan vertical, ladite partie supérieure inclinée de la paroi frontale portant le panneau de commande.
- les moyens de surveillance et de pilotage sont conçus pour et aptes à émettre des signaux d'alarme pour aviser l'utilisateur, en cas de dysfonctionnement de l'unité, ledit dysfonctionnement étant choisi parmi une diminution de la quantité ou de la pression de gaz dans l'une des bouteilles en dessous d'une valeur seuil fixée ; une augmentation de la température de la vanne de la pompe au-dessus d'une valeur de température maximale donnée ; une détection d'au moins une porte en position ouverte ; un défaut de fonctionnement d'au moins une vanne ou d'un capteur agencé sur la ligne de vide ou la ligne de raccordement ; une diminution de la pression de vide en dessous d'une valeur seuil fixée ; une charge électrique de la batterie en dessous d'une valeur seuil fixée ; ou du dépassement d'un délai de maintenance de l'unité.
- les moyens de surveillance et de pilotage sont conçus pour et aptes à émettre des signaux d'alarme sonore et/ou visuel pour aviser l'utilisateur, en cas de dysfonctionnement et/ou à afficher une information de dysfonctionnement sur écran de visualisation.
- en cas de détection de dysfonctionnement, les moyens de surveillance et de pilotage sont conçus pour et aptes à agir sur la pompe de manière à arrêter son fonctionnement, sur une électrovanne agencée en sortie des bouteilles pour fermer ou pour autoriser le passage du gaz dans la ligne de raccordement.

D'autres caractéristiques et avantages de l'unité fonctionnelle et d'assistance de l'invention ressortiront de la description et d'un exemple de réalisation donnés ci-après à titre non limitatif et en références aux figures annexées parmi lesquelles :
- la figure 1 représente une vue en perspective et en élévation verticale de l'unité de secours ou d'assistance selon la présente invention,

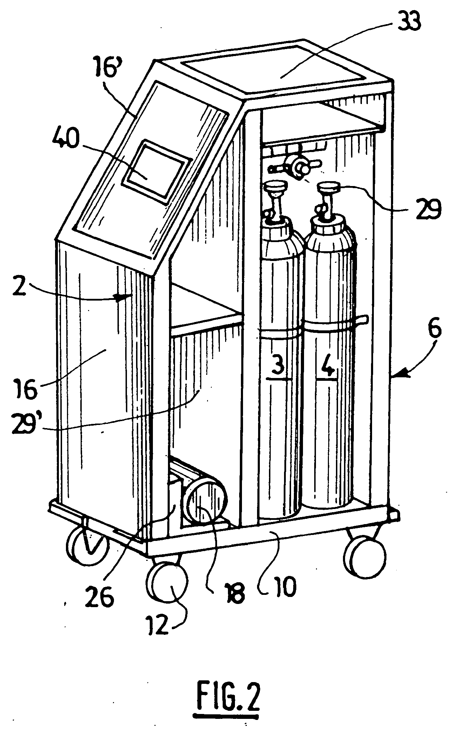

- la figure 2 représente une vue en perspective schématique de l'unité de la figure 1 partiellement ouverte,
- la figure 3 représente une vue latérale schématique de l'unité selon l'invention,
- la figure 4 est une représentation schématique en blocs des composants qui font partie de l'unité de la figure 1,
- la figure 5 représente une vue schématique latérale d'un détail de l'unité fonctionnelle selon la présente invention,
- la figure 6 représente une vue en perspective d'un autre détail de l'arrière de l'unité fonctionnelle selon la présente invention,
- la figure 7 représente une vue schématique d'une unité centrale électronique associée à un écran et à un clavier d'interface avec les utilisateurs de l'unité fonctionnelle selon la présente invention.

Sur les figures, on voit une unité 1 fonctionnelle d'assistance selon la présente invention destinée à l'alimentation d'urgence en gaz médicaux des installations de distribution situées par exemple dans un environnement hospitalier.

L'unité 1 selon la présente invention est d'un type qui comprend au moins une bouteille de réserve de gaz médical, par exemple de l'oxygène, de l'air médical, un mélange He/O₂, un mélange N₂O/O₂ ou tout autre gaz médical.

L'unité 1 comprend une armoire 2 qui loge au moins une paire de bouteilles 3, 4 de gaz médicaux, au moins une prise 5 de prélèvement de gaz qui peut être fixée sur le raccordement rapide des conduits de prélèvement de gaz et associée à une paroi arrière 6 de ladite armoire 2, un raccord d'écoulement 9 entre chaque bouteille 3, 4 de ladite au moins une paire de bouteilles et ladite prise de raccordement 5, et un chariot ou fond 10 supportant ladite armoire 2.

Le chariot 10 est monté sur des roues 12 fixées de manière pivotante et dotées d'un dispositif de blocage, ce qui permet de faciliter le déplacement de l'unité 1 dans les locaux hospitaliers ou analogue, avec un large degré de liberté tout en permettant de la maintenir en position stable, lors de son utilisation. En outre, les angles du chariot 10 sont munis de garnitures angulaires 13 d'amortissement qui protègent soit l'unité 1, soit les personnes ou les objets situés à sa proximité pendant son déplacement et son immobilisation.

L'armoire 2 a de préférence des dimensions compactes, à savoir une hauteur entre 100 et 150 cm environ, par exemple d'environ 144 cm ; une largeur entre 50 et 90 cm environ, par exemple d'environ 71 cm ; et une profondeur entre 70 et 110 cm environ, par exemple d'environ 89 cm. Les dimensions particulièrement compactes et l'extrême facilité de déplacement sur roues 12 rendent l'unité 1 extrêmement commode à utiliser par les opérateurs du secteur hospitalier en cas de situation d'urgence ou même non de routine.

Elle comprend, en outre, une paroi frontale 16 sur laquelle est située un panneau de commande 40, une paroi arrière 6 et deux parois latérales 27, 28. La majeure partie supérieure de la paroi frontale 16 sur laquelle le panneau de commande 40 est logé, est inclinée pour faciliter la vue et la lecture du panneau de commande 40 par l'opérateur.

Une cuvette porte-objets 33 est disposée en creux au sommet de l'armoire 2 et permet de recevoir divers objets ou ustensiles de soin ou analogues.

En outre, deux poignées, par exemple du type à barre 32, sont placées sur chacune des parois latérales 27, 28. Une poignée est également prévue sur la partie arrière 6. Les poignées 32 permettent de fixer les appareils d'urgence au moyen de pinces appropriées. Elles peuvent également être utilisées comme surface de saisie pour déplacer l'unité 1 sur le sol.

L'intérieur de l'armoire 2 définit au moins une paire de compartiments 29 symétriques ou non, dont un seul a été représenté dans les figures, et dans chacun desquels est logée une paire de bouteilles 3, 4 de gaz médical sélectionné, tandis que, pour chaque paire de bouteilles 3, 4, une ou plusieurs prises respectives de prélèvement de gaz 21, 22, 23, 24 sont prévues sur au moins une desdites parois latérales 27, 28 de ladite armoire 2 pour être connectées au raccordement rapide des accessoires de prélèvement de gaz, tels des ventilateurs d'assistance respiratoire ou analogue.

De manière plus particulière, l'unité fonctionnelle 1 comprend au moins une paire de bouteilles 3, 4 pour un premier type de gaz médical, par exemple l'oxygène, et au moins une deuxième paire de bouteilles 7, 8 pour un deuxième type de gaz médical, par exemple l'air de qualité médicale.

Dans un autre mode de réalisation, un autre compartiment 29' accessible par une porte frontale 34 ou latérale 39' est aménagé dans l'armoire 2 et loge une pompe aspirante 18 dont un embout d'aspiration communique à écoulement avec une prise murale 17 pour le vide associée à au moins une des parois latérales 27, 28 de l'armoire 2.

De préférence, les bouteilles de gaz, agencées par paires 3, 4 ou 7, 8, ont une capacité de par exemple 14 litres environ et un diamètre de 171 mm pour une hauteur de 920 mm.

Les deux bouteilles 3, 4 pour l'oxygène et 7, 8 pour l'air médicalisé sont préférentiellement dotées d'une commande automatique de commutation : électrovanne 11 normalement ouverte et commandée par les moyens de pilotage 35.

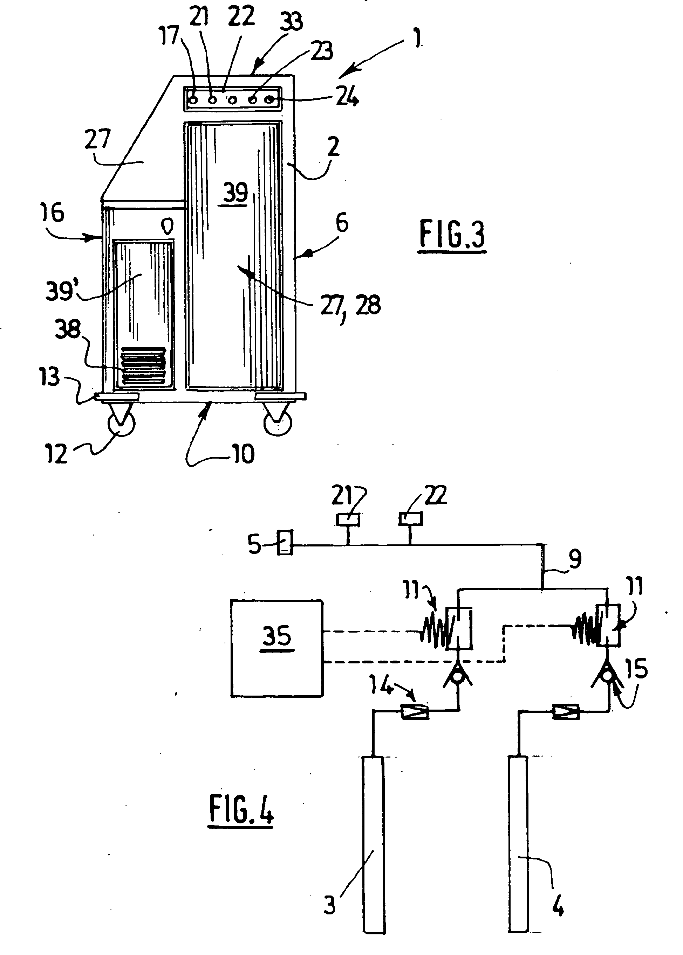

Comme visible en Figure 4, deux électrovannes 11 sont avantageusement prévues sur le raccordement d'écoulement 9 prévu entre chaque bouteille 3, 4 ou 7, 8 de chaque paire de bouteilles et la prise 5 de raccordement correspondante.

De manière encore plus particulière, sur chaque raccordement d'écoulement 9 prévu entre chaque bouteilles d'une paire de bouteilles 3, 4 ou 7, 8 et la prise 5 correspondante de raccordement sont prévus en série : un réducteur de pression 14 doté d'un manomètre et d'un transducteur détendeur de pression, une soupape anti-retour 15 et une électrovanne 11 normalement ouverte et commandée par les moyens de surveillance et de pilotage 35.

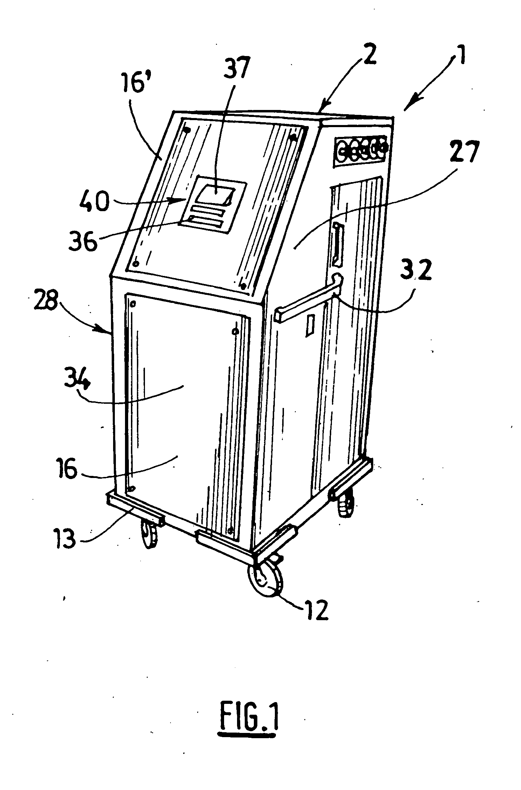

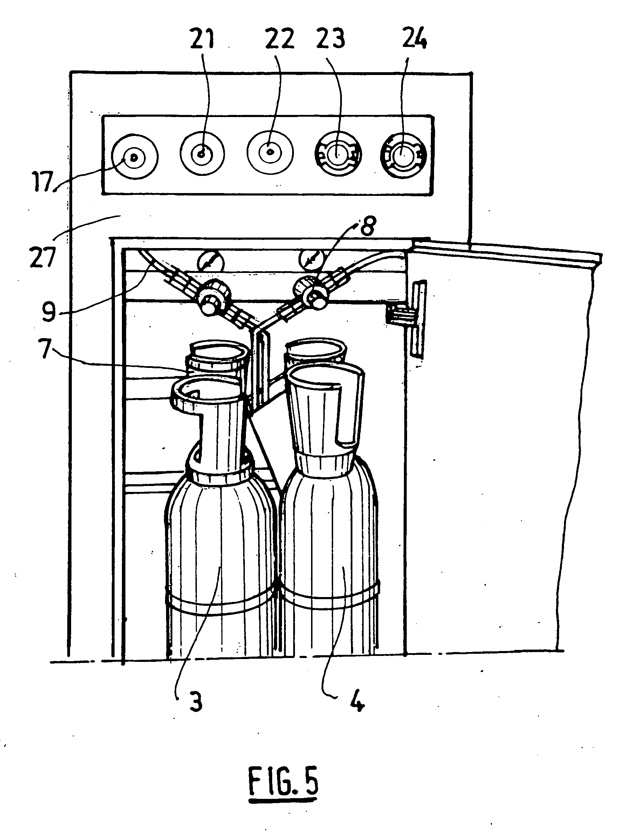

Ainsi que schématisé sur les Figures 1, 3 et 5, l'unité 1 comporte ici 5 prises de prélèvement de gaz, à savoir une prise pour le vide 17, deux prises pour l'oxygène 21, 22 et deux prises pour l'air comprimé 23, 24, à 400 kPa lesquelles sont aménagées à proximité du sommet de chaque paroi latérale 27 , 28 de l'armoire 2.

Ces prises sont préférentiellement de type normalisé, par exemple du type AFNOR NF-S 90-116 ou UNI 9507. Les plaquettes d'identification des prises d'oxygène 21, 22 sont de couleur blanche, tandis que celles prévues pour les prises d'air comprimé 23, 24 se distinguent par une alternance de secteurs circulaires noirs et blancs pour pouvoir les identifier mieux et de manière plus immédiate, et ce conformément aux normes en vigueur. La plaquette d'identification de la prise de vide 17 se distingue à son tour en étant de couleur jaune.

En outre, des prises supplémentaires 5, 25 et 30 de prélèvement de gaz, respectivement pour l'oxygène, l'air comprimé et le vide, sont avantageusement prévues à proximité du sommet de la paroi arrière 6 de l'armoire 2. Ces prises sont aptes à être raccordées facilement et rapidement au réseau de canalisations interne à l'hôpital ou analogue. Les prises supplémentaires 5, 25 sont de type NIST EN 739 pour les gaz médicaux, tandis que la prise de vide 30 est essentiellement formée d'un raccord fileté avec un joint en trois pièces, par exemple d'un diamètre de 19 mm. Les couleurs des plaquettes d'identification correspondent à celles des autres prises prévues sur les parois latérales.

A la différence des prises présentes sur les parois latérales 27 et 28, qui sont prévues pour permettre de prélever les gaz médicaux au moyen de raccords flexibles raccordés à l'appareillage actionné directement par les personnels de santé qui desservent l'unité 1, ces autres prises supplémentaires 5, 25 et 30 sont expressément conçues et adaptées pour être raccordées aux entrées de secours des tableaux de réduction du réseau de distribution à basse pression des gaz médicaux du bâtiment hospitalier, en cas d'urgence provoquée par la perte ou de la chute imprévue de l'alimentation fixe dudit réseau agencé dans le bâtiment hospitalier ou analogue.

Grâce aux prises supplémentaires 5, 25 ou 30, l'unité fonctionnelle 1 devient réellement et intrinsèquement une unité d'assistance destinée à être utilisée en cas de défaillance imprévue de l'alimentation du réseau de distribution des gaz médicaux dans les services hospitaliers, pour garantir la continuité de la distribution par des prises murales situées dans des services relativement éloignées de l'unité 1.

Toutes les prises présentes sur les parois latérales 27, 28 et sur la paroi postérieure 6 de l'armoire 2 sont raccordées à écoulement aux bouteilles respectives de gaz ou à la pompe d'aspiration 18 au moyen de raccordements d'écoulement sur lesquels sont prévus les moyens d'arrêt 14, 15 et 11 précédemment décrits. Les bouteilles 3, 4 ou 7, 8 ont leur propre vanne de fermeture. Un filtre anti-bactériologique est agencé juste avant la pompe pour « éliminer » les impuretés, les bactéries ... (non montré).

Comme montré en figure 4, il faut noter qu'à chaque bouteille de réserve 3, 4 ou 7, 8 sont associés un détendeur de pression 14 et un robinet d'arrêt 15 qui permet de bloquer l'écoulement de gaz lors du remplacement de la bouteille.

Une électrovanne 11 normalement ouverte et pilotée par des moyens de surveillance et de pilotage 35, en particulier une unité électronique centrale , qui sera décrite plus loin est insérée dans le conduit d'écoulement 9 en aval du robinet 15.

L'électrovanne 11 permet d'arrêter automatiquement l'écoulement de gaz d'une des bouteilles et de basculer sur le fluide qui provient de l'autre bouteille. Ceci se fait par commande de l'électrovanne 11 par les moyens de surveillance et de pilotage 35.

Le conduit d'écoulement 9 alimente simultanément les prises installées sur les parois latérales 27, 28 et sur la paroi postérieure 6. Il s'agit de manière plus précise des prises d'oxygène 21, 22 prévues sur les parois latérales 27, des prises correspondantes prévues sur la paroi 28 et de la prise 5 prévue sur la paroi postérieure 6. De manière analogue, pour l'air médical, il s'agit des prises 23, 24 prévues sur la paroi latérale 27, des prises correspondantes prévues sur la paroi 28 et de la prise 25 prévue sur la paroi postérieure 6. Les prises ne peuvent être raccordées à un raccord externe correspondant que l'une après l'autre.

Dans tous les cas, au moins une prise 5 de prélèvement de fluides médicaux gaz est associée à la paroi arrière 6 de l'armoire 2, et est expressément prévue pour assurer la continuité d'alimentation en fluides médicaux l'alimentation par l'installation de distribution centralisée de gaz médical en cas d'urgence provoquée par la perte ou la chute imprévue de l'alimentation fixe de ladite installation de distribution centralisée, en sachant que, pour les gaz médicaux, les normes en cours demandent une mise à disposition sur les unités de seconde détente de prises d'urgences, sur lesquelles l'unité fonctionnelle 1 peut être connectée.

En outre, au moins une prise 23 de prélèvement de gaz est associée à au moins une paroi latérale 27, 28 de l'armoire 2, et est conçue pour permettre de prélever les gaz médicaux au moyen de raccords flexibles, raccordés à l'appareillage, des dispositifs médicaux actionnés directement par actionné directement par les personnels de santé qui desservent l'unité 1.

Pour l'aspiration, la prise de vide 30 est reliée, à l'aide de raccords flexibles, à un autre nouveau dispositif médical installé sur ladite installation de distribution centralisée, à savoir une vanne murale d'arrêt pour aspiration médicale avec entrée d'urgence.

En outre, l'unité fonctionnelle 1 comprend avantageusement des moyens de commande, à savoir une unité centrale électronique 35 de commande agencée dans un compartiment situé en dessous du panneau de commande 40.Les moyens de surveillance et pilotage 35, encore appelés unité centrale électronique 35, comprennent un module d'alimentation, un module d'évaluation et de commande et un module de pilotage proprement dit, ce dernier contenant des composants de puissance qui pilotent chacun des actionneurs électroniques ou électromécaniques de l'unité 1, par exemple les différentes électrovannes 11 ou la pompe 18.

Le module d'alimentation de l'unité centrale électronique 35 est prévu pour réguler l'alimentation électronique de l'unité centrale 35 et de la totalité de l'unité 1. Par exemple, on a prévu ici une alimentation classique via le réseau électrique de 235 V AC +/- 10 %, 50-60 Hz, avec interrupteur à prise bipolaire et terre et porte-fusibles, ainsi qu'une alimentation de réserve ou d'assistance au moyen de batteries électriques embarquées dans l'unité 1 qui sont accessibles par la porte frontale 34.

Plus généralement, l'unité centrale de commande 35 a pour tâche de fournir l'alimentation électrique à tous les composants électriques de l'unité 1, en cas de défaillance de l'alimentation électrique du réseau.

La partie d'évaluation et de commande de l'unité centrale 35 comprend une unité à microprocesseur ainsi que des mémoires classiques à lecture seule ou à lecture et écriture qui permettent à cette unité centrale de fonctionner correctement.

A l'unité centrale électronique 35 sont envoyés des signaux qui proviennent de détecteurs ou capteurs de pression, de température et/ou d'état de marche/arrêt associés aux bouteilles 3, 4, 7 et 8, ainsi qu'à la pompe d'aspiration 18, au logement dans lequel elle se trouve et aux panneaux d'accès des logements. Les autres signaux de commande parviennent à l'unité centrale 35 par l'intermédiaire d'un clavier 36 à disposition des opérateurs , situé sur le tableau de commande 40.

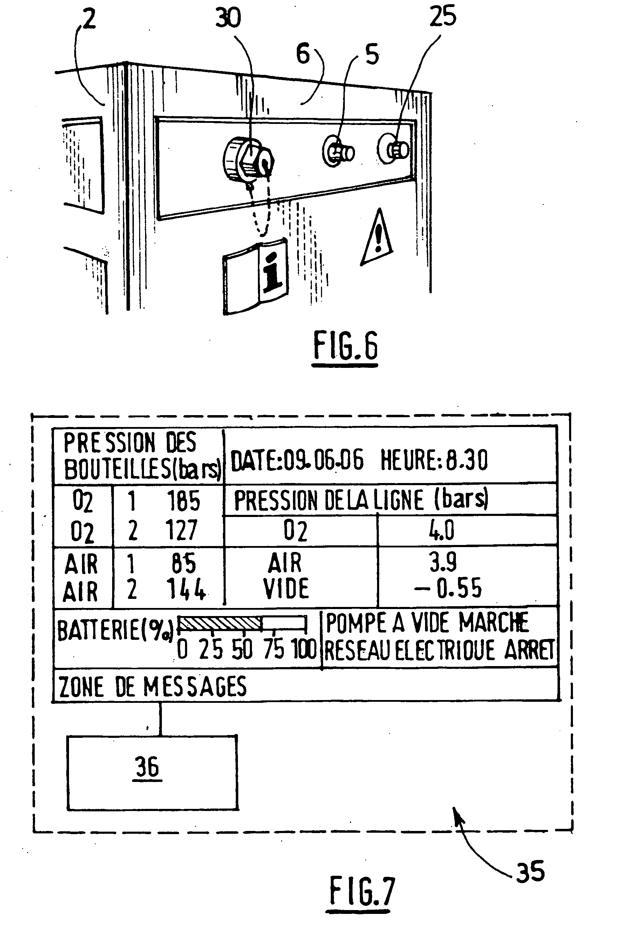

Ainsi qu'on le verra ci-après en référence à la figure 7, en traitant les signaux d'entrée, l'unité centrale 35 est en mesure de présenter sur un affichage graphique 37 à cristaux liquides (LCD) et rétro-éclairé du panneau de commande 40 des informations précises sur l'état de fonctionnement de l'unité 1 et sur la quantité et la pression des gaz présents dans les bouteilles embarquées dans cette unité 1.

Ainsi qu'on l'a déjà dit, l'installation d'aspiration et de création du vide prévue à l'intérieur du compartiment 29 de l'armoire 2 comprend la pompe à vide 18, par exemple un modèle Rietschle VCB20 d'une capacité d'aspiration de 20 m³/h. Une ventilation ou un ventilateur de type 26 à 24 V pour réaliser un échange thermique du compartiment 29, destiné à fonctionner en même temps que la pompe 18, et une sonde thermostatique de contrôle de la température de fonctionnement de la pompe 18 sont également prévues.

Ladite sonde est reliée aux moyens de pilotage 35. De là, lorsqu'elle s'approche de la limite d'une première température de sécurité (+ 50° C), la pompe 18 s'arrête et la ventilation 26 continue à fonctionner jusqu'à ce que le compartiment 29 soit revenu en dessous d'une deuxième température de sécurité (+ 40° C). Dans ce cas, la pompe 18 devra être relancée manuellement pour fonctionner de manière forcée.

Par l'intermédiaire de l'unité centrale électronique 35 de commande, un lancement périodique, par exemple tous les 15 jours, de la pompe 18 est avantageusement programmé quand le module 2 est relié de manière stable au réseau électrique et est en position d'attente et lorsque la machine est à l'arrêt. Cette activation peut durer par exemple un laps de temps préfixé (120 secondes), avec indication correspondante sur l'affichage 37. Par exemple: "vérification de la pompe à vide: n'actionner aucune touche pendant les 120 s qui suivent, un test de fonctionnement va être exécuté".

Pendant cette période consacrée au test, l'unité 1 ne peut pas être utilisée. Pendant la période de test, l'utilisation du chariot 1 a la priorité.

En référence à la figure 7, l'unité centrale électronique 35 permet la gestion de toutes les fonctions de l'unité 1 au moyen du clavier 36 et de l'affichage 37, par exemple, de la manière suivante :
- possibilité de présenter les indications en différentes langues,
- signaux de présence d'un connecteur par émission d'une alarme acoustique,
- apparition du logo et du nom du produit ou de l'entreprise pendant une brève durée préfixée,
- accès à une fonction d'aide, aux données de fabrication et d'installation, sortie,
- indication de la pression des bouteilles (contrôle par l'intermédiaire de transducteur de pression disposé sur le côté Haute Pression du réducteur 14),
- rafraîchissement des chiffres toutes les cinq secondes par exemple,
- gestion de la commutation des bouteilles d'alimentation des circuits d'oxygène et d'air comprimé à 400 kPa,
- état des alarmes (indiqué par l'allumage d'une LED rouge sous la touche déplacée),
- seuils d'entretien des bouteilles : 130 bars - signalisation successive tous les 30 bars avec alarme acoustique pendant 3 s - commutation de la bouteille à 10 bars avec alarme acoustique continue qui peut être éteinte - message spécifique en cas de bouteille épuisée et/ou absente,
- alarme de basse pression de ligne (3,2 bars), peut être coupée ; la durée d'extinction est réglable par l'utilisateur jusqu'à un maximum de 15 minutes,
- alarme de haute pression de ligne (4,8 bars), qui peut être coupée avec période d'extinction réglable,
- alarme de basse dépression (-0,6 bar), qui peut être coupée avec période d'extinction réglable.

Il faut noter que, lorsque l'on atteint une valeur de dépression inférieure à -0,2 pendant plus de 180 s, la pompe s'arrête et devra être relancée manuellement.

En d'autres termes, un logiciel intégré aux moyens de pilotage 35 permet la gestion, le contrôle des alarmes et de l'efficacité de l'unité fonctionelle 1. Ce logiciel est inséré dans l'unité centrale électronique 35 de commande qui reçoit des signaux de commande du clavier 36 aménagé sur l'armoire 2 et qui présente d'autres entrées de signaux reliés à des détecteurs de pression associés à chaque bouteille 3, 4, ladite unité centrale 35 étant raccordée à un afficheur 37 situé sur l'armoire et présentant des sorties de commande pour des actionneurs 11 de blocage du fluide situé sur le (ou les) raccordement ou ligne d'écoulement 9 et pour ladite pompe d'aspiration 18.

L'unité centrale 35 se caractérisant par son aptitude à informer par l'intermédiaire de l'afficheur 37 (cf. figure 7), l'opérateur médical sur l'état de fonctionnement de l'unité de secours, sur la pression résiduelle des bouteilles , sur les débits de distribution des gaz médicaux, et sur la durée résiduelle des gaz contenus dans les paires de bouteilles 3, 4 ou 7, 8, pour les débits sortant plus élevés.

Ce logiciel assure aussi pour, un même gaz, la commutation automatique de la première bouteille « vide » (pression à 10 bar) à la deuxième bouteille pleine.

A noter qu'une vanne d'arrêt peut être installée sur le conduit de vide.

Pour poursuivre avec les fonctions offertes à l'opérateur de l'unité 1, il faut signaler les informations suivantes à disposition de l'utilisateur :
- alarme d'ouverture des portes de service (droite - gauche - centrale), seule l'alarme de la porte centrale permet de commander l'arrêt de la pompe à vide 18,
- erreur électronique ou de logiciel avec signalement du code d'erreur,
- limites de température de la vanne raccordée à la pompe (+40° C / +50° C) avec ensuite commande du verrouillage de la pompe 18,
- approche de la période d'entretien (pompe, filtre, unités terminales) avec signalisation à partir du trentième jour qui précède,
- toutes les alarmes sont visualisées sur la ligne prévue sur l'écran principal, avec séquence d'au moins trois secondes pour chaque alarme et priorité aux fonctions essentielles. En appuyant sur un bouton poussoir (flèche vers le bas), on peut passer à un écran suivant consacré à la visualisation des alarmes.

Après une inactivité d'environ 1 minute, l'unité passe automatiquement à un écran de menu principal. En outre, elle donne également les indications suivantes :
- des différentes pressions et dépressions de ligne,
- état de chargement de la batterie de secours,
- alimentation par le réseau électrique,
- état de fonctionnement de la pompe à vide,
- date et heure,
- nombre de litres effectivement présents dans les bouteilles, indication de la consommation instantanée calculée à partir de la diminution de la pression dans la bouteille toutes les trois minutes pour chaque gaz ainsi que de l'autonomie résiduelle dans les situations plus critiques (acquise à 10 + 10 bars de pression de commutation de chaque bouteille),
- gestion du journal des alarmes, avec indication dans l'écran approprié (les dernières trente alarmes importantes) et déchargement des fichiers par l'intermédiaire d'une prise RS 485 ainsi qu'impression des journaux et/ou des activités de manutention réalisées (état de la machine),
- menu de la partie manutention avec indication des pièces à entretenir, accessible à l'aide du livret d'entretien, par l'intermédiaire d'un mot de passe pour plusieurs opérateurs,
- nécessité de remplacer le filtre (mise à zéro de la durée de fonctionnement) toutes les x heures de fonctionnement et/ou tous les douze mois,
- nécessité de remplacer l'huile de pompe (mise à zéro de la durée de fonctionnement) toutes les x heures de fonctionnement et tous les douze mois,
- nécessité de remplacer le filtre de séparation de la pompe (mise à zéro de la durée de fonctionnement) toutes les x heures de fonctionnement et tous les douze mois,
- nécessité de remplacer le kit de maintenance des prises (mise à zéro de la durée de fonctionnement) tous les douze mois,
- vérification périodique de l'armoire 2 en fonction d'un tableau de fonctionnement et à intervalles réguliers.

De la description qui précède, il ressort nettement que l'unité fonctionnelle selon l'invention permet de pallier aux difficultés de l'art antérieur et d'obtenir d'autres avantages, à savoir notamment :
- Les dimensions particulièrement compactes et l'extrême facilité de déplacement sur roues rendent l'unité 1 extrêmement commode à utiliser par les opérateurs du secteur hospitalier, que ce soit en cas de situation d'urgence ou au cours d'interventions non de routine.
- Les parois latérales de l'armoire 2 sont essentiellement lisses, ce qui revient à dire qu'elles sont hygiéniques et permettent de prévenir les accidents; en outre, le plateau supérieur parfaitement plan est amovible, ce qui permet d'y poser des objets de travail.
- Compte tenu des dimensions limitées, l'encombrement en plan est minimum et, en tout cas aucun espace n'est perdu, puisque l'unité offre un appui utile à la hauteur typique d'un plan de travail.

En pratique, l'unité a une importance vitale pour les patients et pour les opérateurs du secteur qui doivent l'utiliser en cas d'urgence. A cette fin, le chariot est notamment équipé de roues 12 montées de manière pivotante et ses coins sont munis de garnitures d'amortissement de coin 13 pour accroître sa sécurité d'utilisation.

## Revendications

1. Unité fonctionnelle hospitalière (1) mobile pour la distribution momentanée de fluides médicaux, ladite unité étant munie de roues (12) pour permettre un déplacement de l'unité sur le sol et comprenant :
- une armoire (2) comportant une paroi frontale (16), une paroi arrière (6), des parois latérales (27, 28), un sommet (33) et un fond (10), et dont l'intérieur définit au moins un compartiment (29) qui loge une ou plusieurs bouteilles de gaz médical (3, 4 ; 7,8) et au moins une pompe d'aspiration (18) du vide médical, et
- des moyens de surveillance et de pilotage (35) conçus pour et aptes à fournir à un utilisateur au moins une information relative à un dysfonctionnement de l'unité, **caractérisée en ce qu'**elle comprend, en outre :
- au moins une prise arrière (5, 25 ; 30) de prélèvement de fluide médical conçue et adaptée pour être connectée à un réseau de distribution de fluides médicaux agencé au sein d'un bâtiment hospitalier et étant associée à la paroi arrière (6) de ladite armoire (2),
- au moins une prise latérale (21, 22, 23, 24 ; 17) de prélèvement de fluide médical étant associée à au moins une paroi latérale (27, 28) de ladite armoire (2), et
- au moins une ligne de raccordement (9) relie fluidiquement au moins une bouteille de gaz (3, 4 ; 7, 8) à au moins l'une desdites prises arrière (5, 25) et latérale (21, 22, 23, 24) de prélèvement de gaz pour alimenter au moins une desdites prises arrière (5, 25) et latérale (21, 22, 23, 24) avec du gaz délivré par au moins une bouteille de gaz (3, 4 ; 7, 8).

2. Unité selon la revendication 1, **caractérisée en ce qu'**elle comprend au moins deux bouteilles (3, 4), de préférence deux paires de bouteilles de gaz (3, 4 ; 7, 8).

3. Unité selon l'une des revendications 1 ou 2, **caractérisée en ce qu'**elle comprend au moins une paire de bouteilles (3, 4) contenant un premier gaz médical, de préférence de l'oxygène, et au moins une deuxième paire de bouteilles (7, 8) contenant un deuxième gaz médical différent du premier gaz médical, de préférence de l'air médical.

4. Unité selon la revendication précédente, **caractérisée en ce que** les deux bouteilles (3, 4 ; 7, 8) d'une paire de bouteilles de gaz sont raccordées à une ligne de raccordement (9) commune reliant les deux bouteilles de ladite paire de bouteilles à au moins l'une desdites prises arrière (5, 25) et latérale (21, 22, 23, 24) de prélèvement de gaz.

5. Unité selon l'une des revendications 3 ou 4 **caractérisée en ce que** les deux bouteilles (3, 4 ; 7, 8) d'une paire de bouteilles de gaz sont raccordées à une ligne de raccordement (9) commune reliant lesdites deux bouteilles (3, 4 ; 7, 8) de ladite paire de bouteilles à au moins une prise arrière (5, 25) de prélèvement de gaz située sur la paroi arrière (6) de ladite armoire (2), et à au moins une prise latérale (21, 22, 23, 24) de prélèvement de gaz située sur une paroi latérale (27, 28) de ladite armoire (2).

6. Unité selon l'une des revendications précédentes, **caractérisée en ce qu'**une électrovanne (11) est prévue sur la ou chaque ligne de raccordement (9).

7. Unité selon l'une des revendications précédentes, **caractérisée en ce que** au moins une prise arrière (30) et/ou au moins une prise latérale (17) de prélèvement de fluide médical est une prise d'aspiration de vide, et au moins une prise arrière (5, 25) et/ou au moins une prise latérale (21, 22, 23, 24) de prélèvement de fluide médical est une prise de distribution de gaz, en particulier d'oxygène ou d'air médical.

8. Unité selon l'une des revendications précédentes, **caractérisée en ce que**, sur la ou chaque ligne de raccordement (9) sont prévus, agencés en série, entre la ou les bouteilles (3, 4 ; 7, 8) de gaz et la ou les prises de raccordement (5, 25 ; 21-24) correspondantes, un détendeur de pression (14), une soupape anti-retour (15) et une électrovanne (11).

9. Unité selon l'une des revendications précédentes, **caractérisée en ce qu'**au moins une prise arrière (30) et au moins une prise latérale (17) sont des prises d'aspiration de vide reliées fluidiquement, via au moins une ligne de vide, à l'entrée d'aspiration de la pompe d'aspiration (18) de manière à pouvoir créer une dépression au niveau desdites prises.

10. Unité selon l'une des revendications précédentes, **caractérisée en ce qu'**elle comprend au moins une prise latérale de raccordement (21, 22) et au moins une prise arrière de raccordement (5) pour un premier type de gaz médical et au moins une prise latérale de raccordement (23, 24) et au moins une prise arrière de raccordement (25) pour un deuxième type de gaz médical.

11. Unité selon l'une des revendications précédentes, **caractérisée en ce que** les prises de raccordement latérales (21, 22) sont dupliquées sur les deux parois latérales (27, 28) opposées de ladite armoire (2).

12. Unité selon l'une des revendications précédentes, **caractérisée en ce que** la paroi frontale (16) de ladite armoire (2) comporte un panneau de commande (40), de préférence ledit panneau de commande (40) comprend un clavier (36) et/ou un afficheur (37) coopérant avec les moyens de surveillance et de pilotage (35).

13. Unité selon l'une des revendications précédentes, **caractérisée en ce qu'**au moins une des parois latérales (27, 28) comporte une porte (39, 39') donnant accès au compartiment interne (29) contenant la ou les bouteilles ou au compartiment interne (29') contenant la pompe (18), de préférence au moins une porte (39, 39') est aménagée sur chacune des parois latérales (27, 28).

14. Unité selon l'une des revendications précédentes, **caractérisée en ce que** l'intérieur de l'armoire (2) comporte au moins un compartiment (29) principal logeant une ou plusieurs bouteilles de gaz médical (3, 4 ; 7,8) et au moins un compartiment (29') supplémentaire dans lequel est agencée au moins la pompe d'aspiration (18), lesdits compartiments (29, 29') étant munis chacun d'une ou plusieurs portes (39, 39') d'accès, de préférence le compartiment (29') supplémentaire est fermé par une porte (39') munie de fentes d'aération (38).

15. Unité selon l'une des revendications précédentes, **caractérisée en ce qu'**au moins une prise arrière (5, 25 ; 30) de prélèvement de fluide médical gaz est agencée au niveau de l'extrémité supérieure de la paroi arrière (6) et à proximité du sommet (33) et/ou au moins une prise latérale (21, 22, 23, 24 ; 17) de prélèvement de fluide médical est agencée au niveau de l'extrémité supérieure d'au moins une paroi latérale (27, 28) et à proximité du sommet (33).

16. Unité selon l'une des revendications précédentes, **caractérisée en ce qu'**au moins une partie supérieure (16') de la face frontale (16) est inclinée par rapport à un plan vertical, ladite partie supérieure (16') inclinée de la paroi frontale (16) portant le panneau de commande (40).

17. Unité selon l'une des revendications précédentes, **caractérisée en ce que** les moyens de surveillance et de pilotage (35) sont conçus pour et aptes à émettre des signaux d'alarme pour aviser l'utilisateur, en cas de dysfonctionnement de l'unité (1), ledit dysfonctionnement étant choisi parmi une diminution de la quantité ou de la pression de gaz dans l'une des bouteilles (3, 4) en dessous d'une valeur seuil fixée ; une augmentation de la température de la vanne de la pompe au-dessus d'une valeur de température maximale donnée ; une détection d'au moins une porte (39, 39') en position ouverte ; un défaut de fonctionnement d'au moins une vanne ou d'un capteur agencé sur la ligne de vide ou la ligne de raccordement (9) ; une diminution de la pression de vide en dessous d'une valeur seuil fixée ; une charge électrique de la batterie en dessous d'une valeur seuil fixée ; ou du dépassement d'un délai de maintenance de l'unité.

18. Unité selon l'une des revendications précédentes, **caractérisée en ce que** les moyens de surveillance et de pilotage (35) sont conçus pour et aptes à émettre des signaux d'alarme sonore et/ou visuel pour aviser l'utilisateur, en cas de dysfonctionnement et/ou à afficher une information de dysfonctionnement sur écran de visualisation (37).

19. Unité selon l'une des revendications précédentes, **caractérisée en ce qu'**en cas de détection de dysfonctionnement, les moyens de surveillance et de pilotage (35) sont conçus pour et aptes à agir sur la pompe (18) de manière à arrêter son fonctionnement, sur une électrovanne (11) agencée en sortie des bouteilles (3, 4) pour fermer ou pour autoriser le passage du gaz dans la ligne de raccordement (9).

## Claims

1. Mobile functional hospital unit (1) for temporarily distributing medical fluids, the said unit being fitted with wheels (12) so that the unit can be moved around over the floor, and comprising:
- a cabinet (2) comprising a front wall (16), a rear wall (6), side walls (27, 28), a top (33) and a bottom (10), and the interior of which defines at least one compartment (29) which houses one or more medical gas cylinders (3, 4; 7,8) and at least one medical vacuum suction pump (18), and
- monitoring and control means (35) designed and able to provide a user with at least one item of information relating to a malfunctioning of the unit, **characterized in that** it further comprises:
- at least one rear outlet (5, 25; 30) for tapping off medical fluid designed and able to be connected to a medical fluid distribution network arranged within a hospital building and associated with the rear wall (6) of the said cabinet (2),
- at least one side outlet (21, 22, 23, 24; 17) for tapping off medical fluid being associated with at least one side wall (27, 28) of the said cabinet (2), and
- at least one connecting line (9) fluidically connects at least one gas cylinder (3, 4; 7, 8) to at least one of the said rear (5, 25) and side (21, 22, 23, 24) gas tapping outlets in order to supply at least one of the said rear (5, 25) and side (21, 22, 23, 24) outlets with gas delivered by at least one gas cylinder (3, 4; 7, 8).

2. Unit according to Claim 1, **characterized in that** it comprises at least two cylinders (3, 4), preferably two pairs of gas cylinders (3, 4; 7, 8).

3. Unit according to one of Claims 1 and 2, **characterized in that** it comprises at least one pair of cylinders (3, 4) containing a first medical gas, preferably oxygen, and at least one second pair of cylinders (7, 8) containing a second medical gas different from the first medical gas, preferably medical air.

4. Unit according to the preceding claim, **characterized in that** the two cylinders (3, 4; 7, 8) of a pair of gas cylinders are connected to a common connecting line (9) connecting the two cylinders of the said pair of cylinders to at least one of the said rear (5, 25) and side (21, 22, 23, 24) gas tapping outlets.

5. Unit according to one of Claims 3 and 4, **characterized in that** the two cylinders (3, 4; 7, 8) of a pair of gas cylinders are connected to a common connecting line (9) connecting the said two cylinders (3, 4; 7, 8) of the said pair of cylinders to at least one rear gas tapping outlet (5, 25) situated on the rear wall (6) of the said cabinet (2) and to at least one side gas tapping outlet (21, 22, 23, 24) situated on the side wall (27, 28) of the said cabinet (2).

6. Unit according to one of the preceding claims, **characterized in that** a solenoid valve (11) is provided on the or each connecting line (9).

7. Unit according to one of the preceding claims, **characterized in that** at least one rear outlet (30) and/or at least one side outlet (17) for tapping off medical fluid is a vacuum suction outlet and at least one rear outlet (5, 25) and/or at least one side outlet (21, 22, 23, 24) for tapping off medical fluid is an outlet for distributing gas, particularly oxygen or medical air.

8. Unit according to one of the preceding claims, **characterized in that**, on the or each connecting line (9) there are, in series between the gas cylinder or cylinders (3, 4; 7, 8) and the corresponding connecting outlet or outlets (5, 25; 21-24), a pressure regulator (14), a non-return valve (15) and a solenoid valve (11).

9. Unit according to one of the preceding claims, **characterized in that** at least one rear outlet (30) and at least one side outlet (17) are vacuum suction outlets fluidically connected, via at least one vacuum line, to the suction intake of the suction pump (18) so that a partial vacuum can be created at the said outlets.

10. Unit according to one of the preceding claims, **characterized in that** it comprises at least one side connecting outlet (21, 22) and at least one rear connecting outlet (5) for a first type of medical gas and at least one side connecting outlet (23, 24) and at least one rear connecting outlet (25) for a second type of medical gas.

11. Unit according to one of the preceding claims, **characterized in that** the side connecting outlets (21, 22) are duplicated on the two opposite side walls (27, 28) of the said cabinet (2).

12. Unit according to one of the preceding claims, **characterized in that** the front wall (16) of the said cabinet (2) comprises a control panel (40), and the said control panel (40) preferably comprises a keypad (36) and/or a display (37) collaborating with the monitoring and control means (35).

13. Unit according to one of the preceding claims, **characterized in that** at least one of the side walls (27, 28) has a door (39, 39') providing access to the internal compartment (29) containing the cylinder or cylinders or to the internal compartment (29') containing the pump (18), and at least one door (39, 39') is preferably provided on each of the side walls (27, 28).

14. Unit according to one of the preceding claims, **characterized in that** the interior of the cabinet (2) comprises at least one main compartment (29) housing one or more medical gas cylinders (3, 4; 7,8) and at least one additional compartment (29') in which at least the suction pump (18) is positioned, the said compartments (29, 29') each being fitted with one or more access doors (39, 39') and the additional compartment (29') is preferably closed by a door (39') that has ventilation slots (38).

15. Unit according to one of the preceding claims, **characterized in that** at least one rear outlet (5, 25; 30) for tapping off medical gas fluid is positioned at the upper end of the rear wall (6) and near the top (33) and/or at least one side outlet (21, 22, 23, 24; 17) for tapping off medical fluid is positioned at the upper end of at least one side wall (27, 28) and near the top (33).

16. Unit according to one of the preceding claims, **characterized in that** at least an upper part (16') of the front face (16) is inclined with respect to a vertical plane, the said inclined upper part (16') of the front wall (16) bearing the control panel (40).

17. Unit according to one of the preceding claims, **characterized in that** the monitoring and control means (35) are designed and able to transmit alarm signals in order to alert the user to any malfunctioning of the unit (1), the said malfunctioning being chosen among a reduction in the quantity or pressure of gas in one of the cylinders (3, 4) below a fixed threshold value; an increase in the temperature of the pump valve above a given maximum temperature value; detection that at least one door (39, 39') is open; defective operation of at least one valve or sensor positioned on the vacuum line or on the connecting line (9); a reduction in the vacuum pressure below a fixed threshold value; a battery charge below a fixed threshold value; or the fact that the unit is overdue a service.

18. Unit according to one of the preceding claims, **characterized in that** the monitoring and control means (35) are designed and able to transmit audible and/or visible alarm signals to alert the user to any malfunctioning and/or to display information about the malfunction on the display screen (37).

19. Unit according to one of the preceding claims, **characterized in that** if a malfunction is detected, the monitoring and control means (35) are designed and able to act on the pump (18) in such a way as to stop it running, to act on a solenoid valve (11) positioned on the outlet side of the cylinders (3, 4) in order to close this valve, or to allow the gas to pass into the connecting line (9).

## Patentansprüche

1. Mobile Krankenhausfunktionseinheit (1) zur vorübergehenden Verteilung von medizinischen Fluiden, wobei die Einheit mit Rädern (12) versehen, ist, um ein Verschieben der Einheit über den Boden zu gestatten, umfassend:
- einen Schrank (2), der eine Vorderwand (16), eine Rückwand (6), Seitenwände (27, 29), eine Oberseite (33) und einen Boden (10) aufweist und dessen Innenraum mindestens eine Kammer (29) definiert, in der eine oder mehrere Flaschen mit medizinischem Gas (3, 4; 7, 8) und mindestens eine medizinische Vakuumsaugpumpe (18) untergebracht sind, und
- Mittel (35) zur Überwachung und Steuerung, die dazu konzipiert und in der Lage ist, mindestens eine Information, die eine Funktionsstörung der Einheit betrifft, an einen Benutzer zu liefern,
**dadurch gekennzeichnet, dass** sie des Weiteren Folgendes umfasst:
- mindestens einen Hinteranschluss (5, 25; 30) zur Entnahme von medizinischem Fluid, der dazu konzipiert und in der Lage ist, mit einem Netz zur Verteilung medizinischer Fluide verbunden zu werden, das in einem Krankenhausgebäude angeordnet und der Rückwand (6) des Schranks (2) zugeordnet ist,
- mindestens einen Seitenanschluss (21, 22, 23, 24; 17) zur Entnahme von medizinischem Fluid, der mindestens einer Seitenwand (27, 28) des Schranks (2) zugeordnet ist, und
- mindestens eine Anschlussleitung (9), die mindestens eine Gasflasche (3, 4; 7, 8) mit mindestens einem der Hinteranschlüsse (5, 25) und Seitenanschlüsse (21, 22, 23, 24) zur Gasentnahme strömungsverbindet, um mindestens einen der Hinteranschlüsse (5, 25) und Seitenanschlüsse (21, 22, 23, 24) mit durch mindestens eine Gasflasche (3, 4; 7, 8) abgegebenem Gas zu versorgen.

2. Einheit nach Anspruch 1, **dadurch gekennzeichnet, dass** sie mindestens zwei Flaschen (3, 4), vorzugsweise zwei Paare von Gasflaschen (3, 4; 7, 8), aufweist.

3. Einheit nach Anspruch 1 oder 2, **dadurch gekennzeichnet, dass** sie mindestens ein Paar Flaschen (3, 4), die ein erstes medizinisches Gas, vorzugsweise Sauerstoff, enthalten, und mindestens ein zweites Paar Flaschen (7, 8), die ein zweites medizinisches Gas enthalten, das sich von dem ersten medizinischen Gas unterscheidet, aufweist.

4. Einheit nach dem vorhergehenden Anspruch, **dadurch gekennzeichnet, dass** die beiden Flaschen (3, 4; 7, 8) eines Paars Gasflaschen mit einer gemeinsamen Verbindungsleitung (9) verbunden sind, die die beiden Flaschen des Paars Flaschen mit mindestens einem der Hinteranschlüsse (5, 25) und Seitenanschlüsse (21, 22, 23, 24) zur Entnahme von Gas verbindet.

5. Einheit nach Anspruch 3 oder 4, **dadurch gekennzeichnet, dass** die beiden Flaschen (3, 4; 7, 8) eines Paars Gasflaschen mit einer gemeinsamen Verbindungsleitung (9) verbunden sind, die die beiden Flaschen (3, 4; 7, 8) des Paars Flaschen mit mindestens einem Hinteranschluss (5, 25) zur Entnahme von Gas, der sich an der Rückwand (6) des Schranks (2) befindet, und mit mindestens einem Seitenanschluss (21, 22, 23, 24) zur Entnahme von Gas, der sich an der Seitenwand (27, 28) des Schranks (2) befindet, verbindet.

6. Einheit nach einem der vorhergehenden Ansprüche, **dadurch gekennzeichnet, dass** ein Magnetventil (11) an der oder jeder Verbindungsleitung (9) vorgesehen ist.

7. Einheit nach einem der vorhergehenden Ansprüche, **dadurch gekennzeichnet, dass** mindestens ein Hinteranschluss (30) und/oder mindestens ein Seitenanschluss (17) zur Entnahme von medizinischem Fluid ein Vakuumsauganschluss ist, und mindestens ein Hinteranschluss 85, 25) und/oder mindestens ein Seitenanschluss (21, 22, 23, 24) zur Entnahme von medizinischem Fluid ein Anschluss zur Abgabe von Gas, insbesondere Sauerstoff oder medizinische Luft, ist.

8. Einheit nach einem der vorhergehenden Ansprüche, **dadurch gekennzeichnet, dass** an der oder jeder Verbindungsleitung (9) zwischen der oder den Gasflaschen (3, 4; 7, 8) und dem oder den entsprechenden Verbindungsanschlüssen (5, 25; 21 - 24), ein Druckminderer (15), ein Rückschlagventil (15) und ein Magnetventil (11) in Reihe geschaltet vorgesehen sind.

9. Einheit nach einem der vorhergehenden Ansprüche, **dadurch gekennzeichnet, dass** mindestens ein Hinteranschluss (30) und mindestens ein Seitenanschluss (17) Vakuumsauganschlüsse sind, die über mindestens eine Vakuumleitung mit dem Saugeingang der Saugpumpe (18) verbunden sind, um an den Anschlüssen einen Unterdruck erzeugen zu können.

10. Einheit nach einem der vorhergehenden Ansprüche, **dadurch gekennzeichnet, dass** sie mindestens einen Seitenverbindungsanschluss (21, 22) und mindestens einen Hinterverbindungsanschluss (5) für eine erste Art von medizinischem Gas und mindestens einen Seitenverbindungsanschluss (23, 24) und mindestens einen Hinterverbindungsanschluss (25) für eine zweite Art von medizinischem Gas aufweist.

11. Einheit nach einem der vorhergehenden Ansprüche, **dadurch gekennzeichnet, dass** die Seitenverbindungsanschlüsse (21, 22) an den beiden einander gegenüberliegenden Seitenwänden (27, 28) dupliziert sind.

12. Einheit nach einem der vorhergehenden Ansprüche, **dadurch gekennzeichnet, dass** die Vorderwand (16) des Schranks (2) eine Schalttafel (40) aufweist, und die Schalttafel (40) vorzugsweise ein Tastenfeld (36) und/oder eine Anzeige (37) aufweist, die mit den Mitteln (35) zur Überwachung und Steuerung zusammenwirken.

13. Einheit nach einem der vorhergehenden Ansprüche, **dadurch gekennzeichnet, dass** mindestens eine der Seitenwände (27, 28) eine Tür (39, 39') aufweist, die Zugang zu der inneren Kammer (29) gewährt, die die Flasche(n) enthält, oder zu der inneren Kammer (29'), die die Pumpe (18) enthält, und vorzugsweise mindestens eine Tür (39, 39') an jeder der Seitenwände (27, 28) ausgebildet ist.

14. Einheit nach einem der vorhergehenden Ansprüche, **dadurch gekennzeichnet, dass** der Innenraum des Schranks (2) mindestens eine Hauptkammer (29), in der eine oder mehrere medizinische Gasflaschen (3, 4; 7, 8) untergebracht sind, und mindestens eine zusätzliche Kammer (29'), in der mindestens die Saugpumpe 818) angeordnet ist, aufweist, wobei die Kammern (29, 29') jeweils mit einer oder mehreren Zugangstüren (39, 39') versehen sind, und vorzugsweise die zusätzliche Kammer (29') durch eine mit Lüftungsschlitzen (38) versehene Tür (39') geschlossen ist.

15. Einheit nach einem der vorhergehenden Ansprüche, **dadurch gekennzeichnet, dass** mindestens ein Hinteranschluss (5, 25; 30) zur Entnahme von medizinischem gasförmigem Fluid an dem oberen Ende der Rückwand (6) und in der Nähe der Oberseite (33) angeordnet ist und/oder mindestens ein Seitenanschluss (21, 22, 23, 24; 17) zur Entnahme von medizinischem Fluid an dem oberen Ende mindestens einer Seitenwand (27, 28) und in der Nähe der Oberseite (33) angeordnet ist.

16. Einheit nach einem der vorhergehenden Ansprüche, **dadurch gekennzeichnet, dass** ein oberer Teil (16') der Vorderseite (16) bezüglich einer vertikalen Ebene geneigt ist und der geneigte obere Teil (16') der Vorderwand (16) die Schalttafel (40) trägt.

17. Einheit nach einem der vorhergehenden Ansprüche, **dadurch gekennzeichnet, dass** die Mittel (35) zur Überwachung und Steuerung dazu konzipiert und in der Lage sind, Alarmsignale abzugeben, um den Benutzer bei einer Funktionsstörung der Einheit (1) zu benachrichtigen, wobei die Funktionsstörung unter einer Verminderung der Qualität oder des Druckes von Gas in einer der Flaschen (3, 4) unter einem festgelegten Schwellwert; einer Erhöhung der Temperatur des Ventils der Pumpe über einem gegebenen Temperaturhöchstwert; einer Erfassung mindestens einer Tür (39, 39') in geöffneter Stellung; einem Funktionsfehler mindestens eines Ventils oder eines an der Vakuumleitung oder der Verbindungsleitung (9) angeordneten Sensors; einer Verringerung des Vakuumdrucks unter einem festgelegten Schwellwert; einer elektrischen Ladung der Batterie unter einem festgelegten Schwellwert oder einer überfälligen Wartung der Einheit ausgewählt ist.

18. Einheit nach einem der vorhergehenden Ansprüche, **dadurch gekennzeichnet, dass** die Mittel (35) zur Überwachung und Steuerung dazu konzipiert und in der Lage sind, akustische und/oder optische Alarmsignale abzugeben, um den Benutzer bei einer Funktionsstörung zu benachrichtigen und/oder eine Information über die Funktionsstörung auf dem Bildschirm anzuzeigen.

19. Einheit nach einem der vorhergehenden Ansprüche, **dadurch gekennzeichnet, dass** bei Erfassung einer Funktionsstörung die Mittel (35) zur Überwachung und Steuerung dazu konzipiert und in der Lage sind, derart auf die Pumpe (18) einzuwirken, dass ihr Betrieb angehalten wird, und auf ein Magnetventil (11), das am Ausgang der Flaschen (3, 4) angeordnet ist, dazu einzuwirken zu schließen oder das Passieren von Gas in der Verbindungsleitung (9) zu gestatten.
